## Europäisches Patentamt
## European Patent Office
## Office européen des brevets

(19)

(11) Numéro de publication : **0 069 016 B1**

(12)

# FASCICULE DE BREVET EUROPÉEN

(45) Date de publication du fascicule du brevet :
**18.09.85**

(51) Int. Cl.⁴ : **C 07 C 37/20**, C 07 C 39/11, C 07 C 37/11

(21) Numéro de dépôt : **82401173.8**

(22) Date de dépôt : **25.06.82**

(54) Procédé de préparation d'alcools orthohydroxybenzyliques.

(30) Priorité : **25.06.81 FR 8112470**

(43) Date de publication de la demande :
**05.01.83 Bulletin 83/01**

(45) Mention de la délivrance du brevet :
**18.09.85 Bulletin 85/38**

(84) Etats contractants désignés :
**AT BE CH DE FR GB IT LI LU NL SE**

(56) Documents cités :
EP-A- 0 000 165
FR-A- 1 270 731
FR-A- 1 515 387
FR-A- 2 372 182
GB-A- 751 845
US-A- 2 858 342
US-A- 2 909 568

(73) Titulaire : **ISOVER SAINT-GOBAIN**
**Les Miroirs 18, avenue d'Alsace**
**F-92400 Courbevoie (FR)**

(72) Inventeur : **Perrin, Robert**
**Le Hameau Villa 5 87 Chemin de L'Indiennerie**
**F-69370 Saint-Didier au Mont d'Or (FR)**
Inventeur : **Fugier, Roger**
**Rue de la Passerelle**
**F-60290 Rantigny (FR)**

(74) Mandataire : **Muller, René et al**
**SAINT-GOBAIN RECHERCHE 39, quai Lucien Lefranc**
**F-93304 Aubervilliers (FR)**

Jouve, 18, rue St-Denis, 75001 Paris, France

## Description

L'invention a pour objet un procédé de préparation d'alcools orthohydroxybenzyliques et plus particulièrement de l'orthométhylolphénol.

Les alcools orthohydroxybenzyliques sont largement utilisés dans des domaines techniques très variés, ainsi l'orthométhylolphénol plus communément désigné sous l'appellation saligénine ou saligénol est un produit que l'on utilise tant pour ses propriétés intrinsèques notamment pharmacologiques, qu'en tant que produit intermédiaire pour la préparation de produits insecticides ou pour la préparation de produits utilisés dans la parfumerie par exemple.

On connaît la préparation de la saligénine à partir de produits naturels comme l'émulsine réagissant sur la salicyne. Ce procédé ne donne pas entièrement satisfaction car le rendement de la réaction est très faible et de l'ordre de 15 %.

On connaît aussi d'après la publication de brevet français 1 328 945, la fabrication de la saligénine par réaction du formaldéhyde avec un métaborate d'aryle. Ce procédé fournit la saligénine avec un rendement de l'ordre de 65 % exprimé par rapport au phénol et au formaldéhyde mis en œuvre dans la réaction. Ce procédé présente des inconvénients, notamment le formaldéhyde non transformé en saligénine est perdu sous forme de sous-produit et ne peut être récupéré à partir de la masse réactionnelle. L'acide borique, bien que non transformé dans le milieu réactionnel, n'est généralement pas non plus récupérable.

On connaît également d'après la publication de brevet européen 0 007 285, un procédé de préparation de la saligénine consistant à faire réagir des esters boriques de phénol avec du formaldéhyde, ces esters boriques de phénol étant obtenus par réaction d'au moins 1,1 mole d'un phénol avec 1 mole d'acide borique. Ce procédé améliore le rendement en saligénine par rapport au phénol transformé et aussi par rapport au formaldéhyde engagé dans la réaction. Cependant, il nécessite l'emploi de solvants tels le benzène, le toluène, le xylène dans la réaction de ce phénol sur l'acide borique, inconvénient qu'on peut trouver également dans la réaction de condensation du formaldéhyde sur le borate d'aryle. L'emploi de ces solvants pose un délicat problème de pollution et de recyclage de ces produits.

On connaît également la fabrication d'hydroxybenzylalcools produits par réaction de condensation du phénol avec du formaldéhyde en présence d'un catalyseur basique tel un hydroxyde alcalin ou alcalinoterreux, par exemple les hydroxydes de sodium, de potassium, de lithium, de calcium. Mais par ce procédé comme décrit par exemple dans le document EP-A-000 165, on obtient un mélange d'orthométhylolphénol et de paraméthylolphénol dont la séparation est délicate et coûteuse.

D'autres publications de brevets décrivent des réactions de condensation phénols/formaldéhyde en présence de catalyseurs divers, généralement en milieu solvant organique ou aqueux.

Ainsi, le brevet FR-A-1 270 731 décrit la préparation de composés phénoliques alcoylés en milieu solvant organique et en présence d'un hydroxyde de métal alcalin en tant que catalyseur. Le brevet US-A-2 909 568 décrit la préparation du 2,4,6-triméthylphénol(métisol) à partir d'un produit intermédiaire, le 2,6-dihydroxyméthyl-4-méthylphénol obtenu à partir du produit réactionnel du p-crésol et d'hydroxyde de sodium en solution aqueuse et du formaldéhyde. Le brevet FR-A-1 515 387 décrit la préparation d'un mélange de trois composés diméthylolés en présence d'un sel métallique sous forme ionique, dans un milieu solvant organique.

L'invention propose un nouveau procédé de préparation sélective d'alcools orthohydroxybenzyliques et notamment de l'orthométhylolphénol ou saligénine, permettant de travailler en milieu condensé, c'est-à-dire initialement en milieu anhydre, sans utilisation de solvant organique ou aqueux, particulièrement économique en énergie, sans résidu toxique et/ou difficilement recyclable.

Conformément à l'invention on fait réagir des phénols avec un aldéhyde et notamment du formaldéhyde, ceci en milieu condensé et en présence d'une quantité catalytique d'un phénate métallique choisi parmi les phénates d'aluminium et de gallium. On peut utiliser par exemple l'hydroxydiphénate d'aluminium, le triphénate d'aluminium ou un phénate de gallium.

De préférence, on utilise un phénate d'aluminium, à base d'un métal bon marché et considéré comme non polluant. Il est particulièrement avantageux d'utiliser le triphénate d'aluminium qui est facile à préparer, notamment juste avant son utilisation dans la réaction entre le phénol et le formaldéhyde.

L'utilisation d'un catalyseur selon l'invention, tel un phénate d'aluminium, conduit à préparer la saligénine avec un rendement de l'ordre de 90 % compté par rapport au phénol consommé.

Sous un des aspects de l'invention, on utilise du phénate d'aluminium en tant que catalyseur pour la production d'orthométhylolphénols à partir de phénols et d'aldéhydes, et plus spécialement du phénol ordinaire et du formaldéhyde, à une concentration d'au moins 0,1 mole de catalyseur pour 100 moles de phénol et à une température comprise généralement entre 50 et 150 °C, plus particulièrement entre 80 et 100 °C.

On connaissait l'utilisation du phénate d'aluminium en tant que catalyseur pour la réaction d'alkylation du phénol en position ortho. Ainsi lorsqu'on fait réagir le phénol avec l'-méthylstyrène en présence de phénate d'aluminium en tant que catalyseur, on obtient principalement de l'o-cumylphénol avec un peu de p-cumylphénol (Angew. Chemie 69, 699-706 (1957) et Zh. Org. Khim. 10, 1974, 2, 359-364).

Des réactions d'alkylation ou de dialkylation du phénol en position ortho sont également citées dans la publication Angew. Chemie 69, 699, 746 (1957) et dans le brevet français 1 422 944.

Néanmoins la production sélective d'orthométhylolphénols par utilisation du phénate d'aluminium est tout à fait inattendue. En effet, le phénate d'aluminium présente un caractère acide et il est bien connu qu'en présence de catalyseurs acides la réaction de condensation de phénols avec des aldéhydes conduit à la formation de polymères tels les résines novolaques.

Le formaldéhyde utilisé dans la fabrication de la saligénine peut se trouver sous la forme de formaldéhyde gazeux ou de paraformaldéhyde. Dans le cas de l'utilisation du formaldéhyde gazeux, la réaction de condensation peut être plus rapide.

L'utilisation du formaldéhyde gazeux ou du paraformaldéhyde entraîne uniquement une modification des conditions opératoires de réaction, sans influer sensiblement sur son rendement. Il est cependant avantageux d'utiliser du formaldéhyde anhydre et non susceptible de fournir de l'eau en cours de réaction car l'eau éventuellement fournie par la dépolymérisation du paraformaldéhyde risque de décomposer au moins partiellement le phénate d'aluminium qui est particulièrement sensible à l'humidité.

Avantageusement, le rapport molaire de la réaction entre le formaldéhyde et le phénol selon l'invention est le rapport stœchiométrique. Avec un tel rapport, le délicat problème de la séparation des produits de la réaction est sinon évité, tout au moins minimisé. De plus, lorsque le rapport formaldéhyde sur phénol est supérieur à 1, et de l'ordre de 2 et plus, la réaction est généralement beaucoup plus longue que lorsque le rapport est stœchiométrique, ceci dans le cas d'utilisation du formaldéhyde sous forme de paraformaldéhyde. En outre avec un rapport molaire largement supérieur au rapport stœchiométrique, le rendement de la réaction est inférieur à celui de la réaction effectuée avec des réactifs pris en quantités stœchiométriques. Un léger excès ou défaut de l'un des composants n'entraîne pas cependant de modifications importantes dans le rendement de la réaction et les conditions opératoires.

Le catalyseur est avantageusement préparé juste avant son utilisation dans la réaction entre le phénol et le formaldéhyde. On évite ainsi une hydrolyse partielle, éventuelle, du catalyseur et par là une perte au moins partielle de son activité catalytique.

De préférence, le catalyseur est préparé dans le milieu réactionnel lui-même, c'est-à-dire dans du phénol pris en excès, cet excès étant ensuite utilisé comme réactif dans la réaction entre le phénol et le formaldéhyde. Tout le phénol employé est ainsi utilisé, soit dans la réaction de formation du catalyseur, soit dans la réaction entre le phénol et le formaldéhyde et l'on évite en outre la séparation du catalyseur du milieu dans lequel il s'est formé.

D'autres avantages et caractéristiques de l'invention apparaîtront dans la description suivante d'exemples.

Exemple 1

Dans un réacteur en verre de 250 ml, muni d'un agitateur, d'un thermomètre, d'une jaquette dans laquelle circule de l'eau à 85 °C, d'un robinet de prélèvement et d'une garde de CaCl$_2$, on porte 1 mole soit 94 g de phénol à une température de 85 °C. On ajoute 1 mole soit 30 g de formaldéhyde sous forme de paraformaldéhyde. Après 30 minutes environ, durant lesquelles le paraformaldéhyde se dépolymérise partiellement, on introduit 0,012 3 mole soit 3,77 g d'une poudre de phénate d'aluminium anhydre préparé au préalable par exemple par introduction directe de tournure d'aluminium dans le phénol à ébullition ou par réaction d'isopropylate d'aluminium sur du phénol.

On laisse la réaction de condensation s'effectuer pendant environ 2 heures et 50 minutes. Sa progression est contrôlée par chromatographie en phase gazeuse ou liquide. Après la période indiquée le mélange contient notamment les composants suivants :

49,8 g de phénol,
53,3 g d'orthométhylolphénol.

L'analyse ne détecte aucune trace de paraméthylolphénol. Le rendement en orthométhylolphénol est de 91,5 % par rapport au phénol consommé.

La séparation des produits de la réaction peut s'effectuer par un procédé d'extraction entre phase aqueuse et organique comme décrit par exemple dans les publications de brevets allemandes 2 729 075 et 2 915 216. On peut encore séparer l'orthométhylolphénol du phénol restant en utilisant la méthode décrite dans ces publications de brevet français 1 328 945 et européen 0 007 285, c'est-à-dire par une complexation de l'orthométhylolphénol sous forme de complexe de borate, puis destruction de ce complexe par saponification, alcoolyse ou hydrolyse.

Exemple 2

Dans un réacteur en verre de 250 ml, muni d'un agitateur, d'une arrivée de gaz neutre, par exemple de l'azote sec, et chauffé par un chauffe ballon, on prépare le phénate d'aluminium en portant un mélange de 30 grammes de phénol et 0,83 gramme de tournure d'aluminium, à 180 °C pendant 30 minutes. Après refroidissement on obtient un mélange cristallisé de 0,030 7 mole de phénate d'aluminium et de 0,227 mole de phénol.

Dans l'appareillage de l'exemple 1, on porte 0,773 mole de phénol à une température de 85 °C. On ajoute 1 mole de formaldéhyde sous forme de paraformaldéhyde. Après 30 minutes environ, on introduit le mélange phénate d'aluminium-phénol, obtenu préalablement. Le milieu réactionnel contient alors 1 mole de phénol.

Après réaction dans les conditions de l'exem-

ple 1, le mélange contient notamment les composants suivants :

    64,0 grammes de phénol,
    36,0 grammes d'orthométhylolphénol.

La sélectivité en orthométhylolphénol est de 90,6 % par rapport au phénol transformé, c'est-à-dire équivalente à celle de l'exemple 1. L'analyse ne détecte aucune trace de paraméthylolphénol.

## Exemple 3

Dans l'appareillage de l'exemple 1, on porte 1 mole de phénol à une température de 85 °C. On ajoute 1 mole de formaldéhyde, sous forme de paraformaldéhyde. Après 30 minutes environ durant lesquelles le paraformaldéhyde se dépolymérise partiellement, on introduit 0,02 mole d'hydroxydiphénate d'aluminium. Après réaction dans les conditions de l'exemple 1, le mélange réactionnel contient notamment les composants suivants :

    66,0 g de phénol,
    35,0 g d'orthométhylolphénol.

L'analyse ne détecte aucune trace de paraméthylolphénol.

L'hydroxydiphénate d'aluminium montre des propriétés de catalyseur semblables à celles du triphénate d'aluminium pour la réaction sélective de formation de l'orthométhylolphénol.

## Exemple 4

Dans l'appareillage de l'exemple 1, on porte 1 mole de phénol à une température de 85 °C. On ajoute 2 moles de formaldéhyde sous forme de paraformaldéhyde. Après 30 minutes environ, on introduit 0,012 mole de triphénate d'aluminium d'anhydre. On laisse la réaction de condensation s'effectuer pendant environ 2 heures et 30 minutes.

Après réaction, le mélange obtenu est analysé par chromatographie. Le mélange contient notamment les composants suivants :

    66,4 g de phénol,
    40,0 g d'orthométhylolphénol.

L'analyse ne détecte aucune trace de paraméthylolphénol.

Le rendement en orthométhylolphénol, inférieur à celui de l'exemple 1, serait dû à la présence d'une certaine quantité d'eau provenant de la dépolymérisation du paraformaldéhyde, laquelle décomposerait partiellement le triphénate d'aluminium.

## Exemple 5

On se place dans les conditions de l'exemple 1 sauf que la température du mélange réactionnel lors de la réaction de condensation est maintenue à 75 °C. Après 2 heures et 50 minutes, l'analyse chromatographique du mélange révèle une quantité d'orthométhylolphénol inférieure à celle obtenue selon l'exemple 1.

Ce résultat est dû à une réaction de condensation plus lente du fait de la température de la réaction, inférieure à celle de l'exemple 1.

## Exemple 6

Dans un réacteur de 500 ml, muni d'un agitateur, d'un thermomètre et d'une jaquette dans laquelle circule de l'eau à 85 °C, d'un robinet de prélèvement et d'une garde de CaCl₂, on porte 1 mole de phénol à une température de 85 °C . On ajoute environ 0,012 mole de triphénate d'aluminium sous forme de poudre. Dans le milieu réactionnel, on introduit du formaldéhyde gazeux préparé simultanément, par chauffage de paraformaldéhyde en suspension dans une huile siliconée :

Après introduction d'1 mole de formaldéhyde gazeux, le milieu réactionnel est analysé par chromatographie en phase liquide. Le mélange contient notamment les composants suivants :

    49,0 g de phénol,
    54,0 g d'orthométhylolphénol.

L'analyse ne détecte aucune trace de paraméthylolphénol.

La sélectivité en orthométhylolphénol est d'environ 91 % par rapport au phénol transformé.

## Revendications

1. Procédé de préparation d'alcools orthohydroxybenzyliques par réaction de phénols avec un aldéhyde, notamment du formaldéhyde, en présence d'un catalyseur, caractérisé en ce que la réaction s'effectue en milieu initialement anhydre, en présence d'un phénate métallique choisi parmi les phénates d'aluminium et de gallium, pris en quantité catalytique.

2. Procédé selon la revendication 1, caractérisé en ce que le catalyseur est un phénate d'aluminium et de préférence le triphénate d'aluminium.

3. Procédé selon une des revendications 1 à 2, caractérisé en ce qu'on utilise au moins 0,1 mole de catalyseur pour 100 moles de phénol.

4. Procédé selon une des revendications 1 à 3, caractérisé en ce que la température de réaction est comprise entre 50 °C et 150 °C et de préférence entre 80 et 100 °C.

5. Procédé selon une des revendications 1 à 4, caractérisé en ce qu'on surveille la réaction par chromatographie en phase liquide ou gazeuse.

6. Procédé selon une des revendications 1 à 5, caractérisé en ce que le catalyseur est préparé juste avant la réaction entre le phénol et le formaldéhyde.

7. Procédé selon une des revendications 1 à 6, caractérisé en ce que le catalyseur est préparé dans du formaldéhyde en excès, cet excès étant ensuite utilisé comme réactif entre le phénol et le formaldéhyde.

8. Procédé selon une des revendications 1 à 7, caractérisé en ce que les réactifs utilisés sont totalement anhydres et n'engendrent pas d'eau en cours de réaction.

9. Procédé selon une des revendications 1 à 8, caractérisé en ce qu'on utilise les réactifs en quantité stœchiométrique.

10. Application du procédé selon une des revendications 1 à 9 à la préparation de l'orthométhylolphénol.

**Claims**

1. A method of preparing orthohydroxybenzyl alcohols by reaction of phenols with an aldehyde, particularly formaldehyde, in the presence of a catalyst, characterised in that the reaction takes place in an initially anhydrous medium in the presence of a metal phenate chosen from the phenates of aluminium and gallium, taken in a catalytic quantity.

2. Method according to Claim 1, characterised in that the catalyst is an aluminium phenate and preferably aluminium triphenate.

3. Method according to one of Claims 1 to 2, characterised in that at least 0.1 mole of catalyst is used per 100 moles phenol.

4. Method according to one of Claims 1 to 3, characterised in that the reaction temperature is comprised between 50 °C and 150 °C and is preferably between 80 and 100 °C.

5. Method according to one of Claims 1 to 4, characterised in that the reaction is supervised by liquid or gaseous phase chromatography.

6. Method according to one of Claims 1 to 5, characterised in that the catalyst is prepared just prior to the reaction between the phenol and the formaldehyde.

7. Method according to one of Claims 1 to 6, characterised in that the catalyst is prepared in an excess of formaldehyde, this excess being then used as a reagent between the phenol and the formaldehyde.

8. Method according to one of Claims 1 to 7, characterised in that the reagents used are totally anhydrous and do not create any water during the course of reaction.

9. Method according to one of Claims 1 to 8, characterised in that the reagents are used in a stoichiometric quantity.

10. Application of the method according to one of Claims 1 to 9 for the preparation of or-thomethylol phenol.

**Patentansprüche**

1. Verfahren zur Herstellung von Orthohydroxybenzylalkoholen durch Reaktion von Phenolen mit einem Aldehyd, insbesondere Formaldehvd in Gegenwart eines Katalysators, dadurch gekennzeichnet, daß man die Reaktion anfangs in einem wasserfreien Milieu in Gegenwart katalytischer Mengen von Aluminium- oder Galliumphenolat als metallischem Phenolat durchführt.

2. Verfahren nach Anspruch 1, dadurch gekennzeichnet, daß man als Katalysator Alumi-niumphenolat, insbesondere Aluminiumtripheno-lat einsetzt.

3. Verfahren nach einem der Ansprüche 1 oder 2, dadurch gekennzeichnet, daß man mindestens 0,1 Mol Katalysator je 100 Mol Phenol einsetzt.

4. Verfahren nach einem der Ansprüche 1 bis 3, dadurch gekennzeichnet, daß man die Reaktion in einem Temperaturbereich von 50-150 °C, insbesondere 80-100 °C durchführt.

5. Verfahren nach einem der Ansprüche 1 bis 4, dadurch gekennzeichnet, daß man die Reaktion durch Flüssig- oder Gaschromatographie überwacht.

6. Verfahren nach einem der Ansprüche 1 bis 5, dadurch gekennzeichnet, daß man den Katalysator unmittelbar vor der Reaktion zwischen dem Phenol und dem Formaldehyd herstellt.

7. Verfahren nach einem der Ansprüche 1 bis 6, dadurch gekennzeichnet, daß man den Katalysator mit Formaldehyd im Überschuß herstellt, wobei dieser Überschuß anschließend als Reagenz zwischen dem Phenol und dem Formaldehyd verwendet wird.

8. Verfahren nach einem der Ansprüche 1 bis 7, dadurch gekennzeichnet, daß man völlig wasserfreie Reagenzien einsetzt, wobei im Verlauf der Reaktion kein Wasser gebildet wird.

9. Verfahren nach einem der Ansprüche 1 bis 8, dadurch gekennzeichnet, daß man die Reagenzien in stöchiometrischen Mengen einsetzt.

10. Verwendung des Verfahrens nach einem der Ansprüche 1 bis 9 zur Herstellung von Ortho-methylolphenol.